# EUROPEAN PATENT SPECIFICATION

(11) **EP 3 423 071 B1**
(45) Date of publication and mention of the grant of the patent: **18.02.2026**
(21) Application number: 17760980.7
(22) Date of filing: 06.03.2017
(51) Int. Cl.: A61K 35/16, A61K 35/32, A61K 35/14, A61K 35/19, A61P 19/02, C12N 5/077

(54) **CHONDROCYTE PROLIFERATION**
CHONDROZYTENPROLIFERATION
PROLIFÉRATION DES CHONDROCYTES

(30) Priority: 04.03.2016 HU 1600180
(43) Date of publication of application: 09.01.2019
(73) Proprietor: Lacerta Technologies Inc., Raleigh, NC 27616-6417 (US)
(72) Inventor: LACZA, Zsombor, 8229 Csopak (HU); HORNYÁK, István, Vörösmarty utca 53 2/1 (HU); SIMON, Melinda, 1094 Budapest (HU); KUTÉN, Olga, 1190 Wien (AT); JEYAKUMAR, Vivek, 3500 Krems an der Donau (AT)
(74) Representative: Svingor, Adam
(86) International application number: PCT/US2017/020926
(87) International publication number: WO 2017/152172

(56) References cited:
- WO-A1-2016/025496
- US-A1- 2007 134 793
- US-A1- 2011 217 773
- US-A1- 2014 227 241
- US-A1- 2014 227 241
- CHI-SHENG CHIEN ET AL: "Incorporation of exudates of human platelet-rich fibrin gel in biodegradable fibrin scaffolds for tissue engineering of cartilage", JOURNAL OF BIOMEDICAL MATERIALS RESEARCH PART B: APPLIED BIOMATERIALS, vol. 100B, no. 4, 25 May 2012 (2012-05-25), pages 948 - 955, XP055111158, ISSN: 1552-4973, DOI: 10.1002/jbm.b.32657
- SU C Y ET AL: "In vitro release of growth factors from platelet-rich fibrin (PRF): a proposal to optimize the clinical applications of PRF", ORAL SURGERY, ORAL MEDICINE, ORAL PATHOLOGY, ORAL RADIOLOGY AND ENDODONTICS, MOSBY-YEAR BOOK, ST. LOUIS, MO, US, vol. 108, no. 1, 1 July 2009 (2009-07-01), pages 56 - 61, XP026434162, ISSN: 1079-2104, [retrieved on 20090517]
- MICHAEL TOFFLER ET AL: "Introducing Choukroun's Platelet Rich Fibrin (prf) To The Reconstructive Surgery Milieu", THE JOURNAL OF IMPLANT & ADVANCED CLINICAL DENTISTRY, vol. 1, no. 6, 1 September 2009 (2009-09-01), pages 21 - 32, XP055634483
- AKEDA ET AL: "Platelet-rich plasma stimulates porcine articular chondrocyte proliferation and matrix biosynthesis", OSTEOARTHRITIS AND CARTILAGE, ELSEVIER, AMSTERDAM, NL, vol. 14, no. 12, 23 November 2006 (2006-11-23), pages 1272 - 1280, XP005742579, ISSN: 1063-4584, DOI: 10.1016/J.JOCA.2006.05.008

## Description

The invention relates to a novel method for increasing proliferation rate of chondrocytes and for the treatment of articular or joint diseases.

### BACKGROUND ART

Osteoarthritis is a degenerative joint disease and the repair of osteochondral defects yet remains a challenge due to its poor regeneration capacity.

The human body's own cartilage is still the best material for lining knee joints. This drives efforts to develop ways of using cells of the same species or the subject's own cells to grow, or re-grow cartilage tissue to replace missing or damaged cartilage. One cell-based replacement technique is called autologous chondrocyte implantation (ACI) or autologous chondrocyte transplantation (ACT).

A review evaluating autologous chondrocyte implantation was published in 2010. The conclusions are that it is an effective treatment for full thickness chondral defects. The evidence does not suggest ACI is superior to other treatments.[ Vasiliadis, H.; Wasiak, J.; Salanti, G. (2010). "Autologous chondrocyte implantation for the treatment of cartilage lesions of the knee: a systematic review of randomized studies". Knee Surgery, Sports Traumatology, Arthroscopy 18 (12): 1645-1655.]

Platelet-rich plasma (PRP), a fraction of plasma that contains high levels of multiple growth factors, is nowadays widely applied in clinical scenarios as a growth factor pool for improving tissue regeneration. Studies into its clinical efficiency are not conclusive and one of the main reasons for this is that different PRP preparations are used, eliciting different responses that cannot be compared. Amable, P. R. et al. [Amable, P. R. et al. Platelet-rich plasma preparation for regenerative medicine: optimization and quantification of cytokines and growth factors. Stem Cell Research & Therapy20134:67] suggested a standardized PRP and the use of PRP in therapies aiming for tissue regeneration, and its content characterization will allow us to understand and improve the clinical outcomes.

These autologous cells are usually expanded *in vitro* before implantation in cell culture medium that contains serum for supporting the proliferation of the cells. The process required increasing the rate of proliferation.

It has been suggested already in 2006 that PRP isolated from autologous blood may be useful as a source of anabolic growth factors for stimulating chondrocytes to engineer cartilage tissue [Akeda K. et al. Platelet-rich plasma stimulates porcine articular chondrocyte proliferation and matrix biosynthesis. OsteoArthritis and Cartilage 2006 14(12) 1272-1280]. The authors have observed that treatment with PRP growth factors did not markedly affect the types of proteoglycans and collagens produced by porcine chondrocytes, suggesting that the cells remained phenotypically stable in the presence of PRP.

Moreover, Fortier, Lisa A et al. [The Effects of Platelet-Rich Plasma on Cartilage: Basic Science and Clinical Application. Operative Techniques in Sports Medicine, 2011 19(3) 154-159] teach that in preclinical animal model studies, PRP slows the progression of osteoarthritis, but there are mixed results after the use of PRP to facilitate the repair of chondral or osteochondral defects. PRP-bone marrow-derived stromal cell constructs aided in the repair of chondral defects and positive results were also shown 1 year after intra-articular injection in patients suffering from knee pain. Although most studies support the clinical use of PRP for the treatment of cartilage injury and joint pain the authors suggest that more extensive testing and reporting seems to be necessary.

Further uncertainties of the process are involved. Proliferation often includes redifferentiation. It would be desirable to have a method, in order to better regulatability of the process, which promotes proliferation of chondrocytes without promoting differentiation.

Mobasheri, Ali [Chondrocyte and mesenchymal stem cell-based therapies for cartilage repair in osteoarthritis and related orthopaedic conditions. Maturitas 78 (2014) 188-198] review the challenges associated with cartilage repair and regeneration using cell-based therapies that use chondrocytes and mesenchymal stem cells (MSCs) and explore common misconceptions associated with cell-based therapy and highlight a few areas for future investigation. The authors, somewhat gloomily conclude that cell-based therapies may be unrealistic options of the osteoarthritic lesions due to their complex geometry, in contrast to the more local focal defects that may be seen in younger (i.e. athletic patients) and suggest that further basic research is needed. Clearly, in many cases cell-based therapies may not be suitable or effective for end-stage OA.

Peterson L et al. [Peterson L et al. Autologous Chondrocyte Implantation : A Long-term Follow-up. Am J Sports Med 2010 38: 1117] report a study which suggests that the clinical and functional outcomes remain high even 10 to 20 years after the implantation.

Chien Chi-Sheng et al [ "Incorporation of exudates of human platelet-rich fibrin gel in biodegradable fibrin scaffolds for tissue engineering of cartilage" Journal of Biomedical Materials Research Part B: Applied Biomaterials, 100B(4) (2012), 948-955] describe a study to assess the incorporation of exudates of human platelet-rich fibrin (hPRF) into biode-gradable fibrin (FB) scaffolds and the effect of the matrix on chondrocyte proliferation and re-differentiation.

Akeda K et al. ["Platelet-rich plasma stimulates porcine articular chondrocyte proliferation and matrix biosynthesis" OsteoArthritis and Cartilage (2006) 14, 1272e1280] suggest a PRP isolated from autologous blood factors for stimulating chondrocytes to engineer cartilage tissue. The collection of the blood is carried out with an anticoagulant and clotting is induced by adding 10% thrombin solution.

The inventors have surprisingly found that SPRF increased the proliferation rate of chondrocytes, preferably chondrocytes in vitro, in particular dedifferentiated chondrocytes. The inventors have surprisingly found that SPRF increased the proliferation rate of dedifferentiated, preferably osteoarthritic chondrocytes to a larger extent than PRP, and also to a larger extent than FCS. The inventors have also found that the two effects, i.e. proliferation and differentiation of chondrocytes can be separated and therefore a more regulated handling of the procedure. The inventors have also noticed that the effect of SPRF obtained from younger donors is stronger than dose obtained from elder donors.

### BRIEF DESCRIPTION OF THE INVENTION

### The invention is defined in the appended claims.

The invention relates to a use of a serum fraction of platelet rich fibrin (SPRF) prepared from whole blood obtained from one or more donor subject, for increasing proliferation rate of chondrocytes in vitro. Preferably, chondrocytes are dedifferentiated. Preferably, in the use of SPRF it does not redifferentiate chondrocytes from the dedifferentiated state. Preferably, SPRF is obtained by
- providing whole blood obtained from the one or more donor subject(s),
- centrifuging whole blood at 1000 to 4000 g (or in the ranges as disclosed herein) for 2 to 20 minutes (or in a range as disclosed herein) within 4 minutes from blood collection, thereby providing a fibrin clot (platelet rich fibrin),
- pressing or squeezing the fibrin clot to obtain the SPRF as an exudate or releasate thereof.

Preferably, said SPRF comprises less bFGF and/or less G-CSF than PRP and wherein said SPRF comprises less pro-inflammatory factors than PRP said pro-inflammatory factor(s) being selected from the group comprising at least IL-6, IL-8, IL-12, TNF-α.

Preferably, the SPRF does not redifferentiate chondrocytes from the dedifferentiated state or provides a lesser extent of redifferentiation than PRP, preferably if measured by the col II/col I gene expression ratio.

The invention also relates to SPFR for use in transplantation or implantation of chondrocytes into a patient in need thereof, wherein said SPRF is an SPRF prepared from whole blood obtained from a donor subject and wherein said SPRF is used for increasing proliferation rate of chondrocytes to be transplanted or implanted to said patient. Preferably, SPRF it does not redifferentiate chondrocytes from the dedifferentiated state or provides a lesser extent of redifferentiation than PRP, preferably if measured by the col II/col I gene expression ratio. Preferably, SPRF does not change the differentiation state of chondrocytes in hypoxia or normoxia.

In a preferred embodiment the patient to be treated is a subject in cartilage failure, osteoarthritis, cartilage damage, cellular matrix linkage rupture, chondrocyte protein synthesis inhibition, and chondrocyte apoptosis, a condition requiring cartilage regeneration in particular in cartilage ulcer, steoarthritis or traumatic cartilage loss, a condition requiring subchondral bone regeneration in osteoarthritis, Ahlbeck's disease or osteochondral lesions.

In a preferred embodiment the SPRF for use according to the invention is obtained by
- providing whole blood obtained from the one or more donor subject(s),
- centrifuging whole blood at 1000 to 4000 g (or in a range as disclosed herein) for 2 to 20 minutes (or in a range as disclosed herein) within 4 minutes from blood collection, thereby providing a fibrin clot (platelet rich fibrin),
- pressing or squeezing the fibrin clot to obtain the SPRF as an exudate or releasate thereof.

In a further preferred embodiment said SPRF comprises less bFGF and/or less G-CSF than PRP and wherein said SPRF comprises less pro-inflammatory factors than PRP said pro-inflammatory factor(s) being selected from the group comprising at least IL-6, IL-8, IL-12, TNF-α.

In a preferred embodiment the SPRF obtained from a donor subject is used for increasing proliferation rate of chondrocytes *in vitro* before transplantation thereof, wherein preferably SPRF is applied in the cell culture in a concentration between 1-25% or 2-20%, preferably 5 to 15%, highly preferably 8 to 12% or in particular about 10%, wherein the percentage of concentration is given in v/v%. The same concentration range may be applied in liquid pharmaceutical preparations or in gel matrix preparations.

Preferably, the patient is a subject in need of articular cartilage repair, preferably articular cartilage repair, e.g. cartilage replacement therapy.

Preferably, the patient is a subject in cartilage failure, osteoarthritis, cartilage damage, cellular matrix linkage rupture, chondrocyte protein synthesis inhibition, and chondrocyte apoptosis.

In an embodiment the chondrocyte transplantation is autologous transplantation.

In a further embodiment the chondrocyte transplantation is heterologous transplantation.

In a preferred embodiment the donor subject is identical with the patient.

In a preferred embodiment the donor subject is different from the patient. Preferably, the age of the donor subject is below 50 years, preferably below 40 years, more preferably below 35 or 30 years. In a preferred embodiment the age of the patient is above 50 years or above 55 years or above 60 years.

In an embodiment the chondrocytes are transplanted into the patient and SPRF is administered to the patient to the same site as chondrocytes.

In an embodiment the SPRF is administered to the patient to the same site as chondrocytes essentially simultaneous with or after chondrocyte transplantation.

Preferably, SPRF is administered to the patient by injection or by matrix assisted transplantation.

In a preferred embodiment, SPRF obtained from a donor subject is used for increasing proliferation rate of chondrocytes *in vitro* before transplantation thereof.

Preferably, the chondrocytes are dedifferentiated.

Preferably, SPRF does not redifferentiate chondrocytes from the dedifferentiated state.

In an embodiment SPRF for use according to the invention is further provided in a pharmaceutical preparation comprising the SPRF and a pharmaceutically acceptable carrier.

Specifically, the pharmaceutical preparation further comprises an additional active substance and/or device to promote chondrocyte proliferation.

Specifically, the a pharmaceutically acceptable carrier is a matrix. If the matrix further promotes of facilitates chondrocyte transplantation is may be considered as an additional active substance. Preferably, the matrix is a hydrogel, a tissue sealant or an active component thereof, e.g. a gellifying agent which forms a hydrogel upon contact with the serum fraction of the invention, a tissue sealant component comprising fibrinogen and/or collagen, and/or a tissue sealant. In an other embodiment the matrix is a glucoseaminoglycane, e.g. hyaluronic acid or hyaluronane.

In a further embodiment the SPRF is provided in a device for introducing SPRF into the site of cartilage repairs. In particular, the device is a solid or semi-solid or gel-like biomaterial suitable for use in humans (resorbable or non-resorbable).

In a further embodiment the device is an application device, in particular a syringe.

The invention also relates to method for increasing proliferation rate of dedifferentiated chondrocytes comprising contacting a serum fraction of platelet rich fibrin (SPRF), said SPRF being prepared from whole blood obtained from one or more donor subject.

Preferably in this method said SPRF is obtained by
- providing whole blood obtained from the one or more donor subject(s),
- centrifuging whole blood at 1000 to 4000 g (or in a range as disclosed herein) for 2 to 20 minutes (or in a range as disclosed herein) within 4 minutes from blood collection, thereby providing a fibrin clot (platelet rich fibrin),
- pressing or squeezing the fibrin clot to obtain the SPRF as an exudate or releasate thereof.

Preferably in this method said SPRF comprises less bFGF and/or less G-CSF than PRP and wherein said SPRF comprises less pro-inflammatory factors than PRP said pro-inflammatory factor(s) being selected from the group comprising at least IL-6, IL-8, IL-12, TNF-α.

In a preferred embodiment said method is a method for treatment of a patient in need of cartilage repair, in particular articular cartilage repair and/or cartilage replacement therapy, preferably articular cartilage repair, or in more particular the patient is a subject in cartilage failure, osteoarthritis, cartilage damage, cellular matrix linkage rupture, chondrocyte protein synthesis inhibition, and chondrocyte apoptosis, a condition requiring cartilage regeneration in particular in cartilage ulcer, steoarthritis or traumatic cartilage loss, a condition requiring subchondral bone regeneration in osteoarthritis, Ahlbeck's disease or osteochondral lesions, wherein the SPRF is administered to said patient to or at the site of cartilage injury wherein SPRF is contacted with the dedifferentiated chondrocytes.

Preferably SPRF is administered to the patient by injection or by matrix assisted (induced) transplantation. Transplantation and implantation is used herein essentially interchangeably in case of implantation the emphasis being on the implantation step if cells are implanted and not on the origin of cells.

In a preferred embodiment said method is a method of transplantation or implantation of chondrocytes into a patient in need thereof, wherein said SPRF is an SPRF prepared from whole blood obtained from a donor subject and wherein said SPRF is contacted with the dedifferentiated chondrocytes to be transplanted or implanted to said patient *in vitro,* to use for increasing proliferation rate of said chondrocytes.

Preferably, the patient is a subject in need of cartilage repair, in particular articular cartilage repair and/or cartilage replacement therapy, preferably articular cartilage repair,
or in more particular the patient is a subject in cartilage failure, osteoarthritis, cartilage damage, cellular matrix linkage rupture, chondrocyte protein synthesis inhibition, and chondrocyte apoptosis, a condition requiring cartilage regeneration in particular in cartilage ulcer, steoarthritis or traumatic cartilage loss, a condition requiring subchondral bone regeneration in osteoarthritis, Ahlbeck's disease or osteochondral lesions.

Preferably, said transplantation or implantation method comprising the steps of
- culturing the condrocytes to proliferate or to expand,
- introducing the proliferated chondrocytes into the patient.

The chondrocyte transplantation may be autologous transplantation or heterologous transplantation. Preferably, said transplantation or implantation method comprises the steps of
- chondrocytes obtained from the donor subject are contacted with the SPRF,
- the condrocytes are cultured to proliferate,
- the proliferated chondrocytes are introduced into the patient.

In heterologous transplantation the donor subject is different from the patient, and preferably the age of the donor subject is below 50 years, preferably below 40 years, more preferably below 35 or 30 years.

In a preferred embodiment the age of the patient is above 50 years or above 55 years or above 60 years.

In an embodiment transplantation is autologous transplantation,
wherein said method comprising the steps of
- chondrocytes obtained from the donor subject are contacted with the SPRF,
- the condrocytes are cultured to proliferate,
- the proliferated chondrocytes are reintroduced into the patient who is identical with the donor subject.

In a preferred embodiment the chondrocytes are transplanted into the patient and SPRF is administered to the patient to the same site as chondrocytes.

Preferably, SPRF is administered to the patient to the same site as chondrocytes essentially simultaneous with or after chondrocyte transplantation.

Preferably, SPRF is administered to the patient by injection or by matrix assisted transplantation.

Preferably, SPRF does not redifferentiate chondrocytes from the dedifferentiated state or provides a lesser extent of redifferentiation than PRP preferably if measured by the col II/col I ratio.

Preferably, upon culturing of the chondrocytes, e.g. in the *in vitro* step, SPRF is applied in the cell culture in a concentration between 1 to 25% or 2-20%, preferably 5 to 15%, highly preferably 8 to 12% or in particular about 10%, wherein the percentage of concentration is given in v/v%.

In an embodiment SPRF for use according to the invention is further provided in a pharmaceutical preparation comprising the SPRF and a pharmaceutically acceptable carrier.

Specifically, the pharmaceutical preparation further comprises an additional active substance and/or device to promote chondrocyte proliferation.

Specifically, the a pharmaceutically acceptable carrier is a matrix. If the matrix further promotes of facilitates chondrocyte transplantation is may be considered as an additional active substance. Preferably, the matrix is a hydrogel, a tissue sealant or an active component thereof, e.g. a gellifying agent which forms a hydrogel upon contact with the serum fraction of the invention, a tissue sealant component comprising fibrinogen and/or collagen, and/or a tissue sealant.

In a further embodiment the SPRF is provided in a device for introducing SPRF into the site of cartilage repairs. In particular, the device is a solid or semi-solid or gel-like biomaterial suitable for use in humans (resorbable or non-resorbable).

In a further embodiment the device is an application device, in particular a syringe.

### BRIEF DESCRIPTION OF THE FIGURES

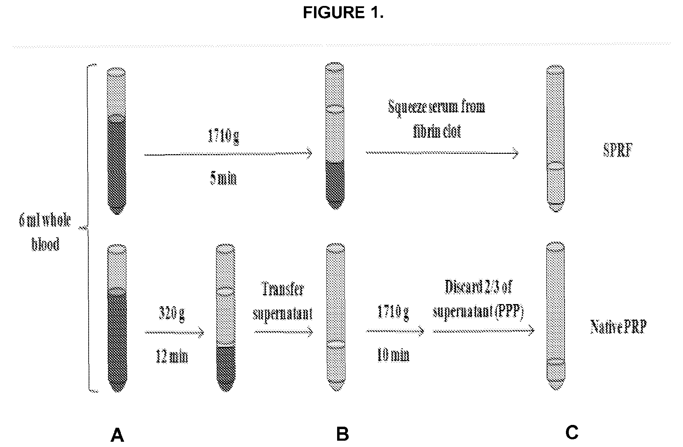

Figure 1. Schematic comparison of exemplary isolation of Platelet rich plasma (PRP) and SPRF.

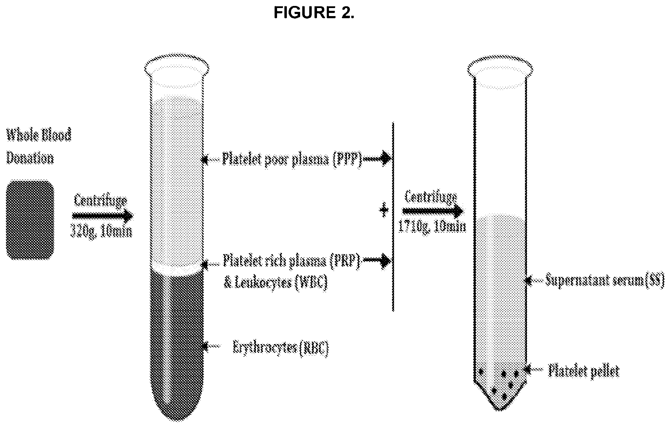

Figure 2. Exemplary isolation of Platelet rich plasma (PRP)
Figure 3. Multiplex array - protein quantification. A. Human blood donors; n=10, data represented as interquartile median. B. Human blood donors; n=6, data represented as interquartile median
Figure 4. Multiplex array - protein quantification. SPRF or PRP pooled from 10 individual donors; data represented as interquartile median.
Figure 5. Cell proliferation of chondrocytes from OA patients.
Figure 6. Col I gene expression of OA chondrocytes under conditions of normoxia and hypoxia.
Figure 7. Col II gene expression of OA chondrocytes under conditions of normoxia and hypoxia.
Figure 8. Index of cell differentiation (redifferentiation). The Col II/Col I ratio is indicative of the cell cell differentiation (in dedifferentiated cell the redifferentiation) potential of cells.
Figure 9. Matrix metalloproteinase 3 (MMP 3) gene expression of OA chondrocytes under conditions of normoxia and hypoxia.
Figure 10. Matrix metalloproteinase 13 (MMP 13) gene expression of OA chondrocytes under conditions of normoxia and hypoxia.
Figure 11. Proliferative effect of serum derivatives in isolated human chondroctytes.
Figure 12. A. Representation of the mean results from 3 different experiments with XTT assay after 8 days, performed in 96-well cell culture plate based monolayer culture with 2000 cells / well seeded, cultured in serum free DMEM (SF) or DMEM media supplemented with 10% SPRF, 10% PRP or 10% FCS. B. Pictures of OA chondrocytes culture after 0,4,8 days with different media supplementation.
Figure 13. Autologous chondrocyte implantation process scheme.

### DEFINITIONS

The term *"administration"* as used herein shall include routes of introducing or applying activated a preparation, such as the serum fraction of the invention, to a subject in need thereof to perform their intended function.

Preferred routes of administration are local, including topical, or application to an injured cartilage site or a site of (surgical) intervention at or near to cartilage, preferably in the form of a fluid, in a hydrogel or collagen matrix or an artificial scaffold (matrix). Specifically preferred are slow-release preparations, e.g. in the form of a hydrogel, a semisolid or solid gel or formulations and delivery systems to provide for the long-acting treatment. Another preferred way of administration is e.g. injection. Various known delivery systems, including syringes, needles, tubing, bags, etc., can be used. Alternative delivery systems employ patches for topical delivery, or implants.

The serum fraction can be administered alone, or in combination or conjunction with either another agent for cartilage repair. The serum fraction can be administered prior to the administration of the other agent, simultaneously with the agent, or after the administration of the agent.

In one embodiment, the serum fraction of the present invention is the only therapeutically active agent administered to a subject, e.g. as a disease modifying or preventing monotherapy.

In another embodiment, the serum fraction of the present invention is combined with further active agents or materials e.g. combined in a mixture or kit of parts.

The serum fraction of the present invention may be administered in combination with one or more other therapeutic or prophylactic active agents or regimens, including but not limited to standard treatment, e.g. antibiotics, steroid and non-steroid inhibitors of inflammation, anti-inflammatory agents, vitamins, or minerals. The term *"subject"* as used herein shall refer to a warm-blooded mammalian, particularly a human being. In particular, the medical use of the invention or the respective method of treatment applies to a subject in need of treatment of a cartilage disorder or disease condition, e.g. associated with damaged cartilage tissue. The term *"patient"* includes human and other mammalian subjects that receive either prophylactic or therapeutic treatment. The term "treatment" is thus meant to include both prophylactic and therapeutic treatment, in particular a treatment including the step of proliferation of chondrocytes.

*"Platelet rich plasma"* (PRP) is a blood fraction prepared by separating the red blood cell fraction from a venous blood sample, removing the red blood cell fraction and, if appropriate, the buffy coat, obtaining thereby a platelet poor plasma fraction (PPP), separating - preferably by centrifugation - a platelet rich fraction from the PPP or pelleting platelets, and recovering the platelets in a platelet rich plasma (PRP) fraction, optionally by resuspending the pelleted platelets in an appropriate medium, optionally in PPP.

Platelet rich fibrin is clotting spontaneously during its preparation by centrifuging a blood sample, preferably accelerated upon contact with negatively charged surfaces and without adding exogenous coagulation activators.

The *"serum fraction of platelet rich fibrin",* hereinafter also referred to as SPRF (serum of platelet rich fibrin) as used herein is isolated from whole blood obtained from donors and is centrifuged at 1000 to 4000 g, preferably 1000 to 3000 g or 1500 to 2500 g or 1000 to 2500 g or 1500 to 2000 g or more preferably 1500 to 2000 g for 2 to 20 minutes or 3 to 15 minutes or 5 to 15 minutes or 3 to 12 minutes or e.g. 5 or 10 mins, within 4, 3, 2 or preferably within 2 or 1.5 or within 1 minute(s) from blood collection, and wherein the serum fraction (fluid fraction) is separated from the fibrin clot (platelet rich fibrin), i.e. a coagel, wherein preferably is pressed or squeezed, to obtain the SPRF as an exudate or a releasate or a fluid fraction of the platelet rich fibrin (PRF). SPRF can be collected and stored e.g. frozen.

Specifically, the fluid fraction of the coagel is separated by pressing, squeezing, filtering and/or centrifuging the coagel to isolate the serum fraction containing the fluid fraction of platelet rich fibrin (PRF).

Preferably, upon clotting and formation of the platelet rich fibrin clot, the acellular or clear supernatant from the PRF may be isolated, or may be removed before fractionating the PRF to isolate the PRF fluid fraction. Such fluid fraction turned out to contain a high concentration of activated platelet releasate and growth factors contained therein.

Preferably, SPRF comprises significantly less bFGF than PRP. Preferably, SPRF comprises less than 100 pg/ml, more preferably less than 50 or 20 pg/ml, highly preferably less than 10 pg/ml, even more preferably less than 5 pg/ml bFGF or essentially comprises no bFGF.

Preferably, SPRF comprises significantly less G-CSF than PRP. Preferably, SPRF comprises less than 100 pg/ml, more preferably less than 50 or 20 pg/ml, highly preferably less than 10 pg/ml G-CSF.

Preferably, SPRF comprises less pro-inflammatory factors than PRP. In particular, SPRF comprises less pro-inflammatory factor than PRP said pro-inflammatory factor(s) being selected from the group comprising at least IL-6, IL-8, IL-12, TNF-α.

Preferably, SPRF comprises significantly less bFGF than PRP.

Preferably, the PRP and SPRF may be obtained from a blood sample from a single donor or from multiple donors and mixed together to obtain a single blood sample. According to a specific aspect, the PRP is obtained from venous blood collected from a single donor.

Preferably, the PRP and SPRF is employed herein without exogenous anticoagulants that are commonly used in the prior art when preparing PRP, thereby an effective activation of platelets and a content of an activated platelet releasate in the isolated serum fraction is obtained according to the invention.

*"Chondrocyte dedifferentiation"* is a process which involves the switching of the cell phenotype towards a state where extracellular matrix production no longer occurs. "Chondrocyte dedifferentiation" is also understood herein as a phenomenon that occurs during chondrocyte expansion in culture on 2D substrates.

*"Chondrocyte redifferentiation"* is a process which involves the switching of the cell phenotype from a dedifferentiated state (e.g. a state obtained by chondrocyte expansion in culture on 2D substrates) into a more differentiated state.

The dedifferentiation or the redifferentiation process can be monitored by differentiation markers e.g. by the Col II/Col I ratio.

*"Dedifferentiated chondrocytes"* as used herein are chondrocytes wherein the Col II/Col I ratio is significantly lower than in healthy control chondrocytes.

In particular, dedifferentiated chondrocytes show reduced extracellular matrix production in comparison with healthy chondrocytes; in particular, dedifferentiated chondrocytes show reduced expression of aggrecan and collagen type II, in particular collagen type IIB in comparison with healthy chondrocytes.

Preferably dedifferentiated chondrocytes are chondrocytes which have been subjected to 2-dimentional culturing and/or cell expansion.

In an embodiment dedifferentiated chondrocytes are arthritic chondrocytes or chondrocytes dedifferentiated due to a cartilage disease or in impaired cartilage.

*"Chondrocyte proliferation"* (or "chondrocyte expansion") is a process which, upon culturing (and expansion) of chondrocytes, involves the propagation or multiplication of the cultured chondrocyte cells.

The term *"comprise(s)"* or *"comprising"* or *"including"* are to be construed herein as having a non-exhaustive meaning and to allow the addition or involvement of further features or method steps or components to anything which comprises the listed features or method steps or components. Such terms can be limited to *"consisting essentially of"* or *"comprising substantially"* which is to be understood as consisting of mandatory features or method steps or components listed in a list, e.g. in a claim, whereas allowing to contain additionally other features or method steps or components which do not materially affect the essential characteristics of the use, method, composition or other subject matter.

As used in this specification and the appended claims, the singular forms *"a",* "an" and *"the"* include plural references, and should be construed as including the meaning *"one or more",* unless the content clearly dictates otherwise. In general, it is also to be understood that the terminology used herein is for the purpose of describing particular embodiments only, and is not intended to be limiting.

### DETAILED DESCRIPTION OF THE INVENTION

Chondrocytes are the main cell type found within cartilage. They are responsible for the synthesis and maintenance of the extracellular matrix (ECM) and are themselves isolated from each other by a large quantity of ECM. Chondrocytes therefore must exist in a low oxygen environment; all this explains the unitary nature of chondrocytes, cartilage and its lo reparative potential and thus the problems arising in clinical conditions like osteoarthritis (OA).

Although articular cartilage can well tolerate physical stress, its ability to heal even a minor injury is particularly low which makes the cartilage tissue particularly sensitive to degenerative processes. Ageing also leads to alterations in ECM composition and alters the activity of the chondrocytes, including their ability to respond to stimuli such as growth factors. Moreover, chondrocytes gradually decline in number with age.

Other typical conditions wherein damage or injury of cartilage occurs are e.g. the following:
Cartilage damage like mechanical cartilage injury, traumatic cartilage loss, cellular matrix linkage rupture, chondrocyte protein synthesis inhibition, chondrocyte apoptosis, cartilage ulcer, subchondral bone damage, Ahlbeck's disease, osteochondral lesions.

In any of these situations the patient may be a subject in need of articular cartilage repair, preferably articular cartilage repair, e.g. cartilage replacement therapy.

Autologous chondrocyte implantation (ACl) is one of the most widely used cell based repair strategies for articular cartilage [Brittberg M et al. Treatment of deep cartilage defects in the knee with autologous chondrocyte transplan- tation. N Engl J Med 1994 331 889-95; Brittberg M. Autologous chondrocyte implantation - technique and long- term follow-up. Injury 2008 39(Suppl 1):540-9.]

In this method cartilage biopsy is taken from the patient from an area which is not weight bearing and preferably which is intact. Then chondrocytes are isolated and transferred into a 2 dimensional culture wherein they are cultured (expanded) to increase their number (multiply them). The so cultured chondrocytes then introduced into the affected (damaged, impaired) area of the cartilage wherein they are fixed.

### Chondrocyte dedifferentiation

The zones of cartilage are based on the shape of the chondrocytes, the composition of the extracellular matrix (ECM) and the orientation of the type II collagen with respect to the articulating surface and the subchondral bone. [Mobasheri, Ali, 2014, infra]. Damaged ECM or its complete absence will result in a major shift in chondrocyte gene expression. Instead of producing cartilage specific proteoglycans and collagen type II, chondrocytes switch to making non-specific proteoglycans and collagen type I.

Chondrocyte dedifferentiation is known to influence cell mechanics leading to alterations in cell function. Dedifferentiation occurs in diseased, e.g. OA cells as well. Typically metabolically active OA chondrocytes no longer express aggrecan and collagen type II. However, chondrocytes in OA cartilage express collagens type I and III, which are rare in normal articular cartilage. OA chondrocytes also express type IIA collagen, a marker of prechondrocyte pheno-type. This expression is enhanced by transforming growth factor-1 (TGF-1). Bone morphogenetic protein-2 (BMP-2), on the other hand, favors the expression of type IIB collagen isoform, a normal component of articular cartilage. Thus, despite their high synthetic activity, dedifferentiated chondrocytes do not express cartilage-specific anabolic genes such as aggrecan or type II collagen, associated with an impairment in anabolic function.

Thus, damaged or ostearthritic chondrocytes show signs of dedifferentiation which is modeled in 2 dimensional chondrocyte cultures used for autologous cell based repair of articular cartilage. While the US Food and Drug Administration has approved the clinical use of chondrocytes that have been expanded *in vitro* to obtain larger number of cells, cells cultured using traditional 2D monolayer conditions undergo dediffierentiation indicated by a phenotypic shift. Dedifferentiated cells are larger, spread and acquire actin stress fibers. They express fibroblastic matrix (such as type I collagen; COL1A1) as well as contractile (alpha smooth muscle actin; aSMA) molecules resulting in biomechanically inferior tissue capable of shrinkage. [Parreno, J, Chondrocyte Dedifferentiation: Actin Regulates the Passaged Cell Phenotype Through MRTFA. Osteoarthritis and Cartilage 22 (2014) S57-S489, abstract].

The term dedifferentiation as used herein includes both dedifferentiation due to a disease process and the process as occurs in vitro, preferably the latter.

In this study, we have investigated the effect of serum from platelet rich fibrin (SPRF) donor age variation on osteoarthritic (OA) chondrocyte culture expansion. SPRF was prepared from 10 individual donors aged 25 to 59 years and added to chondrocyte cultures started from explants harvested at knee replacement surgery.

### Serum from platelet rich fibrin (SPRF)

Preparation of SPRF is also described in US Patent application No. 14/178,573.

Specific method steps applicable in the present invention are e.g. as follows:
1. Obtain whole blood, e.g. venous blood. No additives, e.g. anticoagulants, are necessary.
2. Centrifuge the blood so as to a fibrin clot (also called sometimes a platelet rich fibrin).
3. Obtain SPRF (serum fraction of platelet rich fibrin, a fluid fraction) by pressing or squeezing the fibrin clot (platelet rich fibrin).
4. Storing - Storage is possible in cooled, frozen or freeze-dried forms depending on expected storage time. Preferably, in particular if the SPRF is administered to the patient, it is administered without delay preferably immediately. If the preparation is used for culturing of the cells SPRF may be stored frozen e.g. at -80°C or freeze-dried.

In a preferred embodiment spinning down (centrifugation) is carried out within 20 minutes, preferably within 15, 10 , 5 minutes, or shorter period from obtaining (venous) blood.

Preferably, centrifugation is carried out at 1000 to 5000 g, preferably at 2000 to 4000 g or 1000 g to 3000 g or 1000 to 4000 g, more preferably at about 1200 g to 2500 or at about 1500 to 2000 g. Preferably, centrifugation is carried out for 2 to 20 minutes, preferably for 4 to 15 minutes, highly preferably to about 5 to 12, preferably about 10 minutes (+/- 2 minutes).

The clot obtained (i.e. the coagel) can be removed by any appropriate method, e.g. by filtering or other physical means. In a preferred embodiment continuous centrifugation is applied and the clot is removed at an opening on the wall of the centrifugation space.

The fluid fraction from the clot can be removed by squeezing, pressing, filtering, vacuum filtering or any other appropriate method.

The process can be carried out in an application device e.g. a syringe. Preferably, according to a specific aspect, the serum fraction is freshly prepared and ready- to-use, optionally wherein the serum fraction is provided in the application device. A particular embodiments refers to an autologous serum fraction, i.e. a serum fraction prepared from blood of a single individual donor which is for administration to the same individual. Alternatively, a pooled serum fraction is prepared from multiple patients. In a preferred embodiment the SPRF is prepared from donors of young age, e.g. by donors from 19 to 40 years old e.g. by donors from 20 to 35 years old.

In a preferred embodiment the donor subject is different from the patient. Preferably, the age of the donor subject is below 50 years, preferably below 40 years, more preferably below 35 or 30 years. In a preferred embodiment the age of the patient is above 50 years or above 55 years or above 60 years.

The serum fraction may be conveniently prepared in an appropriate preparation device suitable for aseptic collection of the blood. Negatively charged surfaces are preferred. The isolated serum fraction may be produced in the application device in an aseptic way and may conveniently be directly and immediately administered to the individual, e.g. by an applicator aseptically connected to the preparation device, or by a separate application device or kit which allows the aseptic transfer of the prepared serum fraction to the application device and/or to administer the preparation to the individual.

According to the invention, the serum fraction is specifically provided for use in the manufacturing of an autologous pharmaceutical or medicinal product. Such product may be in the form of a pharmaceutical preparation or a medical device preparation.

Specifically, the serum fraction is provided for the treatment of the serum fraction's donor. Specifically, the autologous use of the serum fraction is preferred.

### Multiplex array

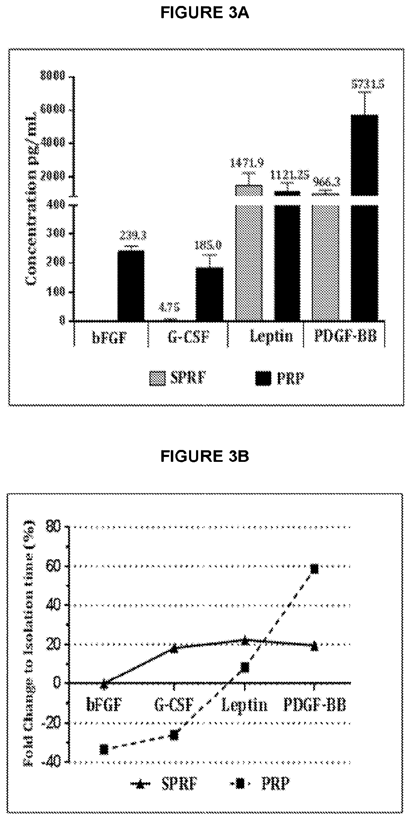

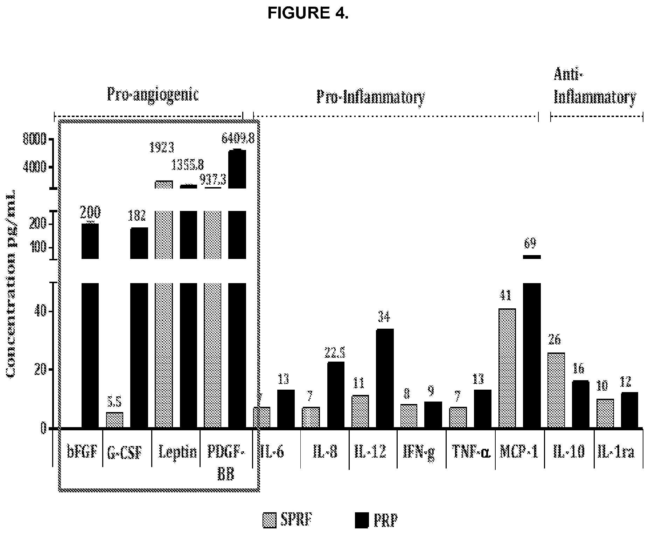

We have performed a multiplex array - protein quantification (Figures 3 to 4) to characterize the fractions.

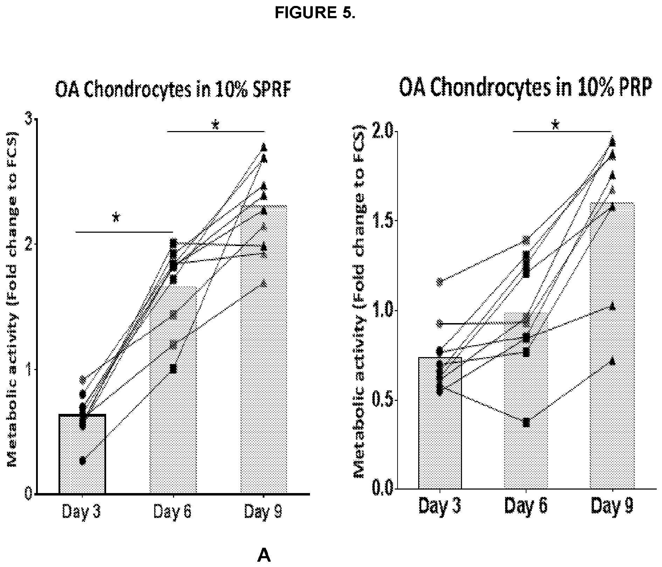

In cell proliferation experiments of chondrocytes from OA patients we have surprisingly seen that SPRF was slighty more effective than PRP in particular from younger donors in promoting proliferation (see e.g. Figure 5).

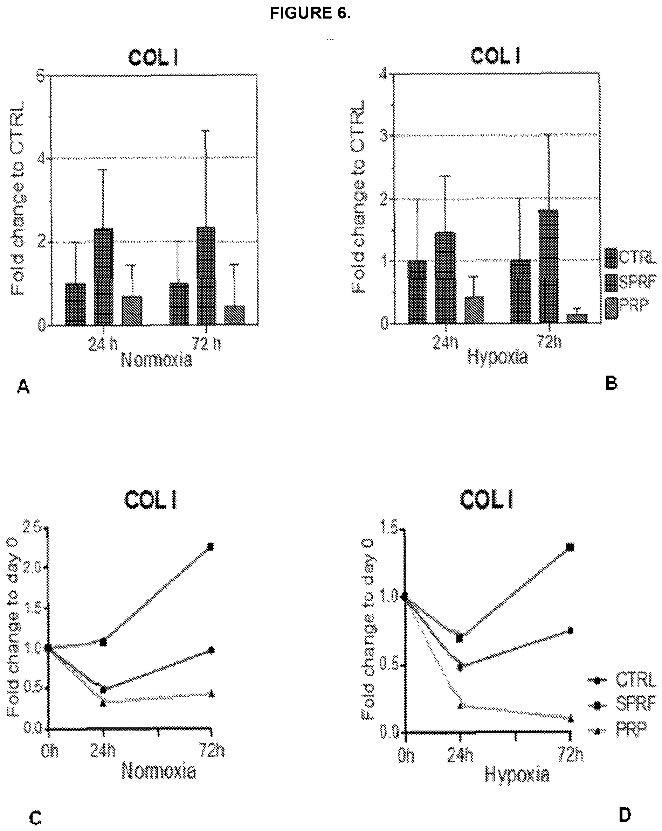

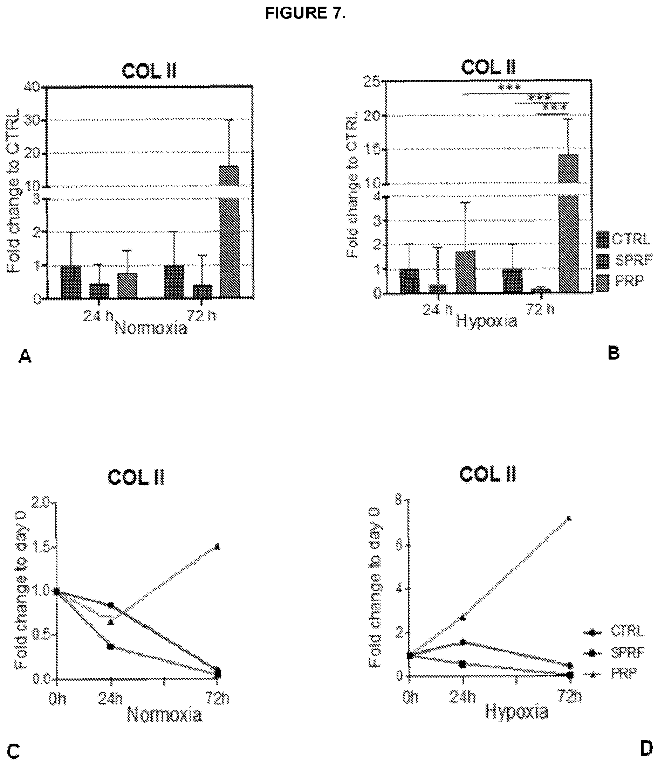

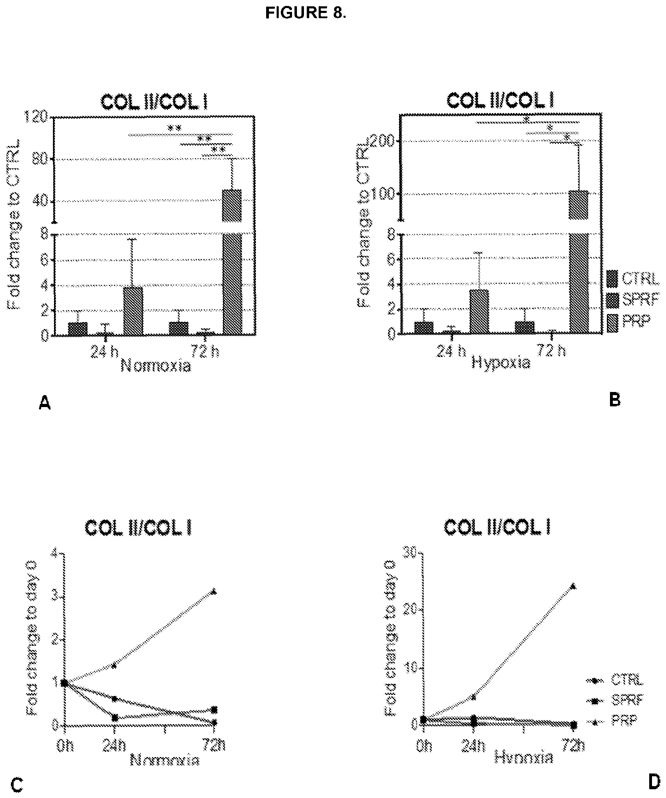

The Col II/Col I ratio is indicative of the cell-cell differentiation (in dedifferentiated cell the redifferentiation) potential of cells. Measurement may be made at the mRNA or protein level. We have shown that this index of differentiation is very low when SPRF is used both under conditions of normoxia and hypoxia (Figures 6 to 8).

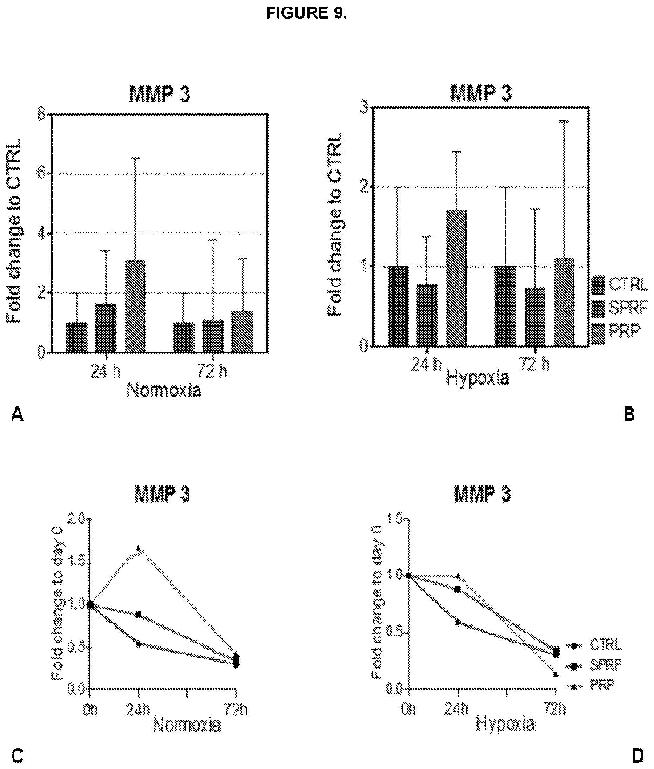

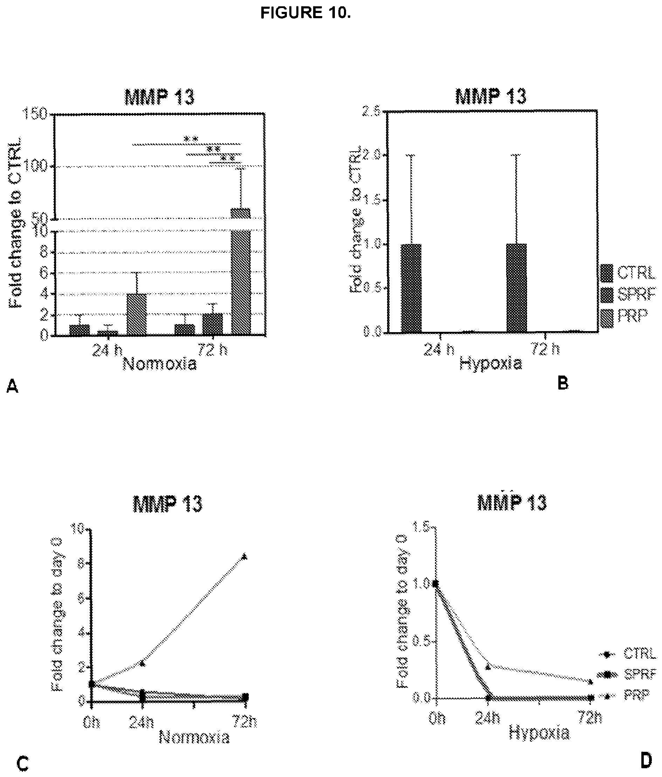

Proteins of the matrix metalloproteinase (MMP) family are involved in the breakdown of extracellular matrix proteins and during tissue remodeling in normal physiological processes, such as embryonic development and reproduction. MMP 3 is involved in normal collagen breakdown. MMP13 is expressed in the skeleton as required for restructuring the collagen matrix for bone mineralization. In pathological situations it is highly overexpressed; this occurs in human carcinomas, rheumatoid arthritis and osteoarthritis.[ Johansson N, Ahonen M, Kähäri VM. (2000). "Matrix metalloproteinases in tumor invasion.". Cell Mol Life Sci. 57 (1): 5-15]. Figures 9 and 10 show a normal expression of these genes under the condition of normoxia. Matrix metalloproteinase 3 (MMP 3) gene expression was normally regulated also under conditions of hypoxia. Matrix metalloproteinase 13 (MMP 13) gene expression was reduced under conditions of hypoxia in case of SPRF and PRP which is normal and suggest that SPRF is safe.

### Chondrocyte transplantation or implantation

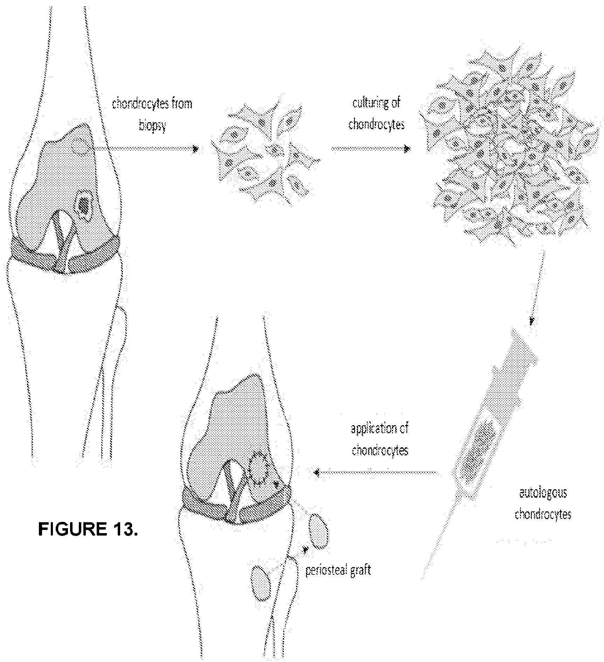

Figure 13 shows an autologous chondrocyte implantation process scheme. Such methods are known e.g. in the literature below, as far as treatment of cartilage with chondrocytes is disclosed. Autologous chondrocyte implantation is described e.g. in Robi et al, Current Issues in Sports and Exercise Medicine (2013) / 978-953-51-1031-6.]. Also, a review is provided by Vasiliadis, H. et al. [Vasiliadis, H.; Wasiak, J.; Salanti, G. (2010). "Autologous chondrocyte implantation for the treatment of cartilage lesions of the knee: a systematic review of randomized studies". Knee Surgery, Sports Traumatology, Arthroscopy 18 (12): 1645-1655.]. A further summary is provided by Mobasheri, Ali et al. [Mobasheri, Ali et al. Chondrocyte and mesenchymal stem cell-based therapies for cartilage repair in osteoarthritis and related orthopaedic conditions. Maturitas 78 (2014) 188-198]. Peterson, Lars et al. [Peterson, Lars et al. Autologous Chondrocyte Implantation : A Long-term Follow-up. The American Journal of Sports Medicine, 38(6) 2010 1117] report on a long term follow of patients treated by first generation ACI and conclude that ACI is an effective and durable solution for the treatment of large full-thickness cartilage and osteochondral lesions of the knee joint. They add that second- and third-generation ACI techniques have been developed since 1987, using either manufactured coverings or scaffolds for the 3-dimensional culturing of the chondrocytes or, in third generation ACI, fully arthroscopical administration can further improve outcomes in the future.

The present inventive methods provided herein seamlessly fit into such scheme by adding SPRF to the chondrocyte culturing medium. SPRF is added to the culturing medium of chondrocytes so as to improve their proliferation rate, preferably *in vitro.* SPRF is in one embodiment simply mixed into the media. Upon implantation of the cells SPRF may be administered simultaneously.

### Autologous chondrocyte implantation with scaffold (matrix) or cover

Variants of the autologous chondrocyte implantation method are methods using a cover for the chondrocyte, e.g. a periosteum-cover technique e.g. wherein type I/type III collagen is used as a cover (ACI-C) and matrix-induced autologous chondrocyte implantation (MACI) using a collagen bilayer seeded with chondrocytes. The methods are described e.g. in Bartlett W. et al. [Bartlett W. et al. Autologous chondrocyte implantation versus matrix-induced autologous chondrocyte implantation for osteochondral defects of the knee. J Bone Joint Surg [Br] 2005;87-B:640-5.]. A more recent report on MACI is provided by Schneider, T., E. and Karaikudi S. [Schneider, T., E., Matrix-Induced Autologous Chondrocyte Implantation (MACI) Grafting for Osteochondral Lesions of the Talus. Foot & Ankle International 30(9) 2009]. The studies report that the MACI technique is a basically reliable treatment method. According to the invention such method can be used with a minimal adaptation wherein the chondrocytes are to be cultured in SPRF.

Upon culturing the chondrocytes the SPRF is preferably used in a concentration of between 1-25% or 2-20%, preferably 5 to 15%, highly preferably 8 to 12% or in particular about 10%, wherein the percentage of concentration is given in v/v%.

### Administration SPRF to the site of cartilage damage.

An alternative method for the treatment of cartilage injuries is the administration of SPRF to the site of cartilage damage. This method should be preceded by a diagnosis of the cartilage injury. Very seriously damaged cartilage may not be successfully treated by this technique. Also, some healthy cartilage may be necessary to be present.

The SPRF is prepared as described herein, and included into an appropriate physiologically acceptable buffer system. In an embodiment the SPRF

### Matrix (scaffold) assisted administration of SPRF

In an embodiment SPRF can be added to a matrix analogously to a matrix-induced autologous chondrocyte implantation matrix, with or without chondrocytes.

Below the invention is further illustrated by non-limiting examples.

### EXAMPLE 1

Serum from platelet-rich fibrin (sPRF) and the platelet-rich plasma (PRP) were obtained from whole blood of 11 donors. Isolated sPRF and PRP will be stored at -80°C as stocks and aliquots for cell-culture experiments and GF, cytokine quantification assays by luminex.

### MATERIALS

### Sterile

▪ Tweezers (sharp and thin tweezer), Sharp scissor
▪ VACUETTE^{®} 9 ml K3 EDTA Blood Collection Tube (REF.no. 455036, Greiner Bio-one)
▪ VACUETTE^{®} 9ml Z Serum C/A (REF.no. 455092, Greiner Bio-one)
▪ Polypropylene falcon tubes 15 ml
▪ Eppendorf tubes 2 ml

### Non-sterile

▪ Centrifuge

### METHOD

### Isolation of SPRF:

a) Whole blood obtained from donors was centrifuged at 1700g for 10 mins at RT in the VACUETTE^{®} 9ml Z Serum C/A.
b) The fibrin clot from the tube was gently removed with a sharp tweezer and placed onto a sterile petri dish and the red blood cells at the bottom of the fibrin clot were cut with a sharp scissor and discarded.
c) Now, using a flat forceps the lysate was squeezed out of the fibrin clot, collected, stored at -80°C.

### Isolation of PRP:

d) Whole blood obtained from donors was centrifuged at 320 g for 12 mins at RT in the VACUETTE^{®} 9 ml K3 EDTA blood collection tube.
e) Three layers had been formed in the collection tube. The bottom layer containing the red blood cells, middle layer containing the buffy coat and the top layer containing the Platelet-Poor plasma (PPP).
f) The top layer (PPP) was aspirated along the middle layer (buffy coat) and transferred into a 15mL falcon tube and centrifuged at 1700g for 10 mins, the pellet was resuspended into an corresponding volume to the isolated SPRF in the supernatant (PPP), stored at -80°C.

### Data from informed consent and parameters for isolation

**Table 1.0 Isolation of SPRF with defined parameters**

| **Donor ID No.** | **Gender** | **Age** | **Total blood collected** | **Volume *tubes centrifuged for SPRF** | **Centrifugation period for SPRF** | **G-Force/RPM** | **Collected serum from PRF** | **Volume stored at -80°C for Stock** | **Volume *vials stored at -80°C for Luminex** |
|---|---|---|---|---|---|---|---|---|---|
| Donor 1 | F | 57 | 36mL | 9mL* 2 | 10 mins | 1710g/3000 | 1 | 1mL | NIL |
| Donor 2 | F | 40 | 36mL | 9mL* 2 | 10 mins | 1710g/3000 | 1.5 | 1.5mL | NIL |
| Donor 3 | F | 24 | 36mL | 9mL* 2 | 10 mins | 1710g/3000 | 0.2 | 0.2mL | NIL |
| Donor 4 | M | 25 | 36mL | 9mL* 2 | 10 mins | 1710g/3000 | 1.8 | 1.8mL | NIL |
| Donor 5 | M | 32 | 36mL | 9mL* 2 | 10 mins | 1710g/3000 | 2.4 | 2.4mL | NIL |
| Donor 6 | M | 34 | 36mL | 9mL* 2 | 10 mins | 1710g/3000 | 2.5 | 2.5mL | NIL |
| Donor 7 | M | 29 | 36mL | 9mL* 2 | 10 mins | 1710g/3000 | 1.1 | 1.1mL | NIL |
| Donor 8 | F | 31 | 36mL | 9mL* 2 | 10 mins | 1710g/3000 | 1 | 1.0mL | NIL |
| Donor 9 | F | 39 | 36mL | 9mL* 2 | 10 mins | 1710g/3000 | 1.05 | 0.8mL | 75ul*2 |
| Donor 10 | M | 30 | 36mL | 9mL* 2 | 10 mins | 1710g/3000 | 2.65 | 2.5mL | 75ul*2 |
| Donor 11 | F | 36 | 36mL | 9mL* 2 | 10 mins | 1710g/3000 | 2.15 | 2.0mL | 75ul*2 |
| Donor 12 | M | 36 | 36mL | 9mL* 2 | 10 mins | 1710g/3000 | 1.45 | 1.3mL | 75ul*2 |
| Donor 13 | F | 58 | 36mL | 9mL* 2 | 10 mins | 1710g/3000 | 1.625 | 1.5mL | 125ul*1 |
| Donor 14 | F | 53 | 36mL | 9mL* 2 | 10 mins | 1710g/3000 | 3.725 | 3.6mL | 125ul*1 |
| Donor 15 | F | 52 | 36mL | 9mL* 2 | 10 mins | 1710g/3000 | 2.425 | 2.3mL | 125ul*1 |
| Donor 16 (not sterile) | F | 58 | 36mL | 9mL* 2 | 10 mins | 1710g/3000 | 1.0 | 0.875mL | 125ul*1 |
| Donor 17 | F | 27 | 36mL | 9mL* 2 | 10 mins | 1710g/3000 | 1.8 | 1.675mL | 125ul*1 |
| Donor 18 | F | 50 | 36mL | 9mL* 2 | 10 mins | 1710g/3000 | 2.3 | 2.175mL | 125ul*1 |
| Donor 19 | M | 57 | 36mL | 9mL* 2 | 10 mins | 1710g/3000 | 2.0 | 1.875mL | 125ul*1 |
| Donor 20 | M | 61 | 36mL | 9mL* 2 | 10 mins | 1710g/3000 | 2.4 | 2.275mL | 125ul*1 |

**Table 2.0 Isolation of PRP with defined parameters**

| **Donor ID No.** | **Gen der** | **Age** | **Total blood collected** | **Volum. *tubes centrifuged for PRP** | **Centrif. period 1 (mins)** | **G-Force/RPM** | **Coll. S. nat. (mL)** | **Centrif. period 2 (mins)** | **G-Force/RPM** | **Vol. stored - 80°C for Stock (mL)** | **Volume* vials stored at -80°C for Luminex** |
|---|---|---|---|---|---|---|---|---|---|---|---|
| Donor 1 | F | 57 | 36mL | 9mL* 2 | 12 | 320/1200 | 4 | 10 | 1710/3000 | 1 | NIL |
| Donor 2 | F | 40 | 36mL | 9mL* 2 | 12 | 320/1200 | 6 | 10 | 1710/3000 | 1.5 | NIL |
| Donor 3 | F | 24 | 36mL | 9mL* 2 | 12 | 320/1200 | 4 | 10 | 1710/3000 | 0.2 | NIL |
| Donor 4 | M | 25 | 36mL | 9mL* 2 | 12 | 320/1200 | 4 | 10 | 1710/3000 | 1.8 | NIL |
| Donor 5 | M | 32 | 36mL | 9mL* 2 | 12 | 320/1200 | 6 | 10 | 1710/3000 | 2.4 | NIL |
| Donor 6 | M | 34 | 36mL | 9mL* 2 | 12 | 320/1200 | 6 | 10 | 1710/3000 | 2.5 | NIL |
| Donor 7 | M | 29 | 36mL | 9mL* 2 | 12 | 320/1200 | 4 | 10 | 1710/3000 | 1.1 | NIL |
| Donor 8 | F | 31 | 36mL | 9mL* 2 | 12 | 320/1200 | 6 | 10 | 1710/3000 | 1 | NIL |
| Donor 9 | F | 39 | 36mL | 9mL* 2 | 12 | 320/1200 | 6 | 10 | 1710/3000 | 0.8 | 75ul*2 |
| Donor 10 | M | 30 | 36mL | 9mL* 2 | 12 | 320/1200 | 6 | 10 | 1710/3000 | 2.5 | 75ul*2 |
| Donor 11 | F | 36 | 36mL | 9mL* 2 | 12 | 320/1200 | 4 | 10 | 1710/3000 | 2.5 | 75ul*2 |
| Donor 12 | M | 36 | 36mL | 9mL* 2 | 12 | 320/1200 | 4 | 10 | 1710/3000 | 1.3 | 75ul*2 |
| Donor 13 | F | 58 | 36mL | 9mL* 2 | 12 | 320/1200 | 4 | 10 | 1710/3000 | 1.5 | 125ul*1 |
| Donor 14 | F | 53 | 36mL | 9mL* 2 | 12 | 320/1200 | 4 | 10 | 1710/3000 | 3.6 | 125ul*1 |
| Donor 15 | F | 52 | 36mL | 9mL* 2 | 12 | 320/1200 | 6 | 10 | 1710/3000 | 2.3 | 125ul*1 |
| Donor 16 (not sterile) | F | 58 | 36mL | 9mL* 2 | 12 | 320/1200 | 6 | 10 | 1710/3000 | 0.875 | 125ul*1 |
| Donor 17 | F | 27 | 36mL | 9mL* 2 | 12 | 320/1200 | 6 | 10 | 1710/3000 | 1.675 | 125ul*1 |
| Donor 18 | F | 50 | 36mL | 9mL* 2 | 12 | 320/1200 | 6 | 10 | 1710/3000 | 2.175mL | 125ul*1 |
| Donor 19 | M | 57 | 36mL | 9mL* 2 | 12 | 320/1200 | 6 | 10 | 1710/3000 | 1.875mL | 125ul*1 |
| Donor 20 | M | 61 | 36mL | 9mL* 2 | 12 | 320/1200 | 6 | 10 | 1710/3000 | 2.275mL | 125ul*1 |

| | | | | | | | | | | | |
|---|---|---|---|---|---|---|---|---|---|---|---|
| *After centrifugation period 2, the pellet (PRP) was re-suspended in an equal amount of PPP (Supernatant) in a volume corresponding to the obtained volume from SPRF | | | | | | | | | | | |

### Observations during experiments

▪ In the preparation of SPRF, it was noted that **clot formation** in a particular donor is **dependent on the time factor;** i.e., Whole blood fractions obtained from donors not centrifuged within 4, 3, 2 or preferably 1 minute(s) or **immediately** resulted in impaired clot formation.
▪ In an example, we tested this observation, by obtaining two vials from a donor, that were centrifuged immediately (less than a minute) and one vial from the same donor, which was centrifuged after 5 minutes. It was observed that the vials centrifuged immediately (within 1 minutes) had a clot formation, whereas the vial centrifuged after 5 mins had no clot formation.
▪ It has also been observed that the amount of **PRF derived serum (quantity) isolated from each donor is donor dependent.** It varies from each individual.

### EXAMPLE 2 - The proliferative effect of serum derivatives on isolated human chondrocytes

### Obtaining and culturing chondrocytes

Human osteoarthritic articular cartilage was obtained from patients (n = 3; age 60-80 years) undergoing total knee arthroplasty. For chondrocyte isolation, articular cartilage was minced into 2 mm3 pieces prior to enzymatic digestion with Liberase Blendzyme 3 (0.2 WU/mL, Roche Diagnostics GmbH, Mannheim, Germany) in medium (GIBCO DMEM/F12 GlutaMAX-I, Invitrogen, LifeTech Austria, Vienna, Austria) with antibiotics (penicillin 200 U/mL; streptomycin 0.2 mg/ mL, and amphotericin B 2.5 µg/mL (Sigma-Aldrich Chemie GmbH, Steinheim, Germany)) under permanent agitation for 18 to 22 hours at 37°C. Subsequently, cell suspensions were passed through a 40 µm filter (BD, Franklin Lakes, NJ) to remove debris, washed with phosphate-buffered saline (PBS), centrifuged (10 minutes, 500 × g, room temperature) and resuspended in PBS. Cells were seeded into 75 cm2 culture flasks (Nunc, Rochester, NY) at a density of ≈100 cells/cm2 and further cultivated in medium supplemented with antibiotics (see above), 10% fetal calf serum (PAA Laboratories GmbH, Linz, Austria), and I-ascorbic acid (50 µg/mL; Sigma- Aldrich Chemie GmbH, Steinheim, Germany) at 37°C in a humid environment with 5% CO2. Medium was changed every 3 days. For passaging, cells grown to 80% confluency were harvested by use of accutase (1.5 mL/flask; PAA Laboratories GmbH, Linz, Austria) counted, and seeded again (all experiments were performed in passage 1)

### Proliferation assay

OA chondrocytes (passage 1) were seeded in three 96-well plate (2000 cells/well) For obtaining reliable results, cells for every tested group were seeded in 20 wells (in few different rows placed on the 96- well plate - to eliminate any disruptions in the data that could be associated with the possible mistake in pipetting, resulting in differences with the number of cells in wells) - and for each group 12 wells should remain without cells only with media (to check the absorbance of medium). After seeding the cells were fed for 48 hours with 100 µl of standard growing medium (described above). 48 hours after seeding (day 0) standard growing medium was replaced with 100 µl of following growth medium per well : group with **SPRF** - DMEM medium (GIBCO^{®}DMEM/F12 GlutaMAX^{™}-I, Invitrogen, Vienna, Austria) with 10% SPRF, 2% Penicilin/Streptomycin, 1% Amphotericin; group with **PRP** - DMEM medium (GIBCO^{®}DMEM/F12 GlutaMAX^{™}-I, Invitrogen, Vienna, Austria) with 10% PRP, 2% Penicilin/Streptomycin, 1% Amphotericin, Heparine (2 U/ml); **FCS** group : standard growing medium as described above; **serum free group (SF)** : DMEM medium (GIBCO^{®}DMEM/F12 GlutaMAX^{™}-I, Invitrogen, Vienna, Austria) with 2% Penicilin/Streptomycin, 1% Amphotericin. Experiments lasted 8 days and measurements were taken at three different time points (days 0, 4, 8 - 1 plate was used at one time point). Metabolic activity and proliferation rate of OA chondrocytes were investigated through XTT assay (Roche, Mainheim, Germany) according to the manufacturer's instructions. Relative fluorescence was measured using plate reader The BioTek's Synergy 2.

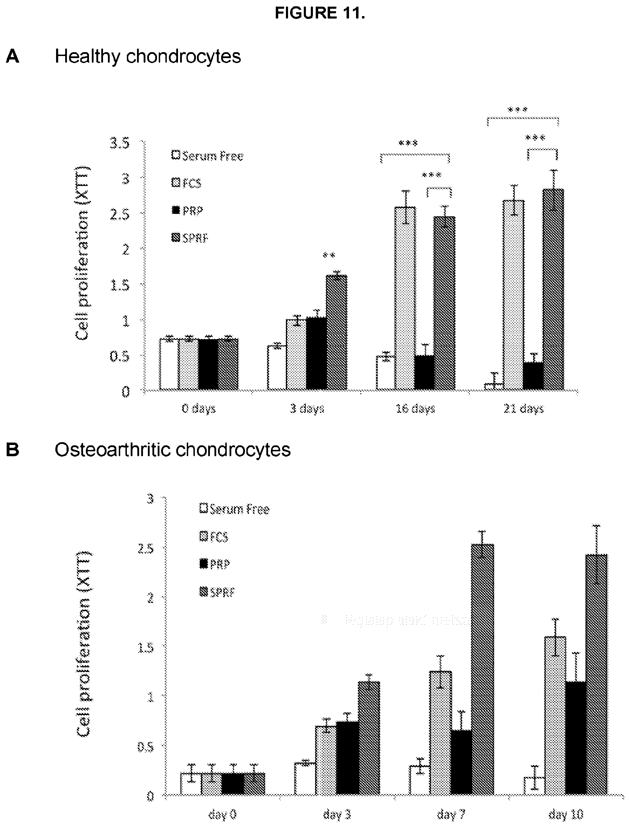

In a preliminary experimente, figure 11, Panels A and B the proliferative effect of serum derivatives is shown in isolated human chondrocytes.

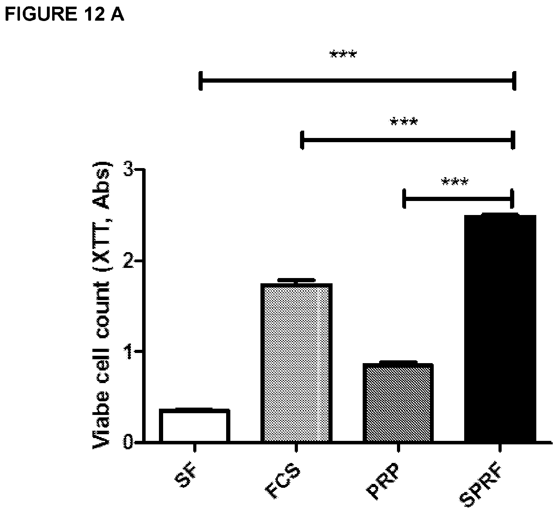

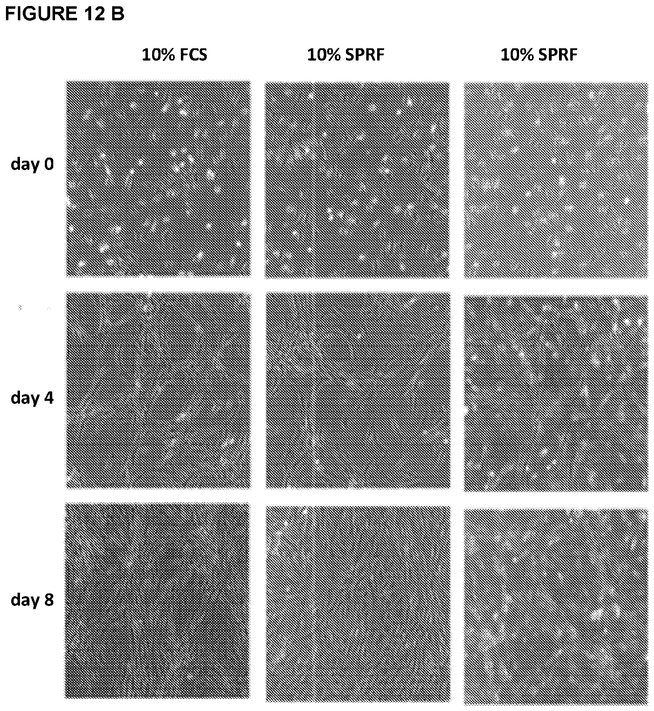

On Figure 12, panel A the mean results from 3 different experiments with XTT assay after 8 days is shown, performed in 96-well cell culture plate based monolayer culture with 2000 cells / well seeded, cultured in serum free DMEM (SF) or DMEM media supplemented with 10% SPRF, 10% PRP or 10% FCS. On panel B pictures of OA chondrocytes culture after 0,4,8 days with different media supplementation are shown.

Cells were harvested from either healthy or osteoarthritic human knee hyalin cartilage and grown in culture. In terms of proliferation support, FCS and SPRF are comparably effective, while PRP has no effect in healthy chondroytes. In osteoarthritic chondrocytes the differences are even more pronounced, SPRF outperforms FCS and PR shows some effect.

### EXAMPLE 3

### SPRF preparation in a syringe ready for intraarticular injection

18 mL blood (without anticoagulant) from the right or left forearm vein of the patient is taken using e hypodermic needle (e.g. butterfly needle) into glass or silica-coated tubes then spun in a centrifuge at 1714G for 8 min (5-10 min). The red blood cell fraction is removed and the remaining yellowish clotted plasma (=platelet rich fibrin, PRF) is gently squeezed to harvest the SprF fraction. Alternatively, SPRF can be prepared in the hypACT Inject Auto medical device using the same method. The SPRF fraction is then transferred into a standard 5 mL syringe ready for intraarticular injection.

### EXAMPLE 4

### Autologous Chondrocyte Implantation

It is confirmed that the lesion is eligible for autologous chondrocyte implantation. Then a biopsy is taken from the cartilage and is sent for chondrocyte culturing (cell proliferation) in the laboratory. Chondrocyte proliferation is carried out as described above.

Cell implantation is performed in the following stage, consisting of arthrotomy, preparation of the chondral defect, harvesting of periosteum, hermetic fixation of periosteum over the lesion with stitches and fibrin glue, injection of chondrocyte concentrate and closing of the operative wound.

In a further variant, second generation ACI is applied, i.e. after cell expansion in a monolayer, the cells are deposited on a carrier membrane/matrix, obtaining a membrane sown with MACI^{®} (chondrocytes (Verigen AG, Leverkusen, Germany).

In a further variant of the example, a third generation of ACI is applied, i.e the chondrocyte culture is deposited on a matrix of hyaluronic acid structured in three dimensions (Hyalograft-C^{®}, Fidia Advanced Biopolymers, Abano Term, Italy), thus enabling homogeneous distribution of the chondrocytes inside the lesion.

### Conclusions

SPRF increases the proliferation rate of OA chondrocytes as observed with PRP on 2D substrates but is very donor dependent. However, SPRF does not redifferentiate the OA chondrocyte from the dedifferentiated state either under normoxic and hypoxic (physiological) conditions.

However, PRP enhances proliferation & redifferentiation both under normoxic and hypoxic conditions from 24h to 72h.

Moreover, as an example, over a culture period of 9 days SPRF from younger donors (± 35 years) reached a higher proliferation rate compared to older donors (± 55 years). In contrast to the biological activity, growth factor concentrations (PDGF-BB, Leptin) were not age-dependent in the SPRF preparations. However, the growth factor concentrations of individual SPRF donors measured at two different time points were highly variable as quantified with a multiplex screening array. Our results indicate that SPRF from younger donors expedite proliferation of OA chondrocytes derived from older patients and can be a relevant serum replacement during cell culture expansion or in vivo therapy.

We have also found that FCS does not retain the redifferentiated state (Col2/Col1 differentiation index) under normoxic/hypoxic conditions from 24h to 72h.

### INDUSTRIAL APPLICABILITY

The present invention is applicable both in research and medicine among others to improve proliferation of chondrocytes without affecting their differentiation status.

## Claims

1. Use of a serum fraction of platelet rich fibrin (SPRF) prepared from whole blood obtained from one or more donor subject, for increasing proliferation rate of dedifferentiated chondrocytes *in vitro,*
wherein said SPRF provides a lesser extent of redifferentiation of chondrocytes from the dedifferentiated state than platelet-rich plasma (PRP), wherein said SPRF is obtained, without exogenous anticoagulants, by a method comprising the steps of
- providing whole blood obtained from the one or more donor subject(s),
- centrifuging whole blood at 1000 to 4000 g for 2 to 20 minutes within 4 minutes from blood collection, thereby providing a fibrin clot (platelet rich fibrin), clotting spontaneously during its preparation by centrifuging,
- pressing or squeezing the fibrin clot to obtain the SPRF as an exudate or releasate thereof.

2. The use of SPRF according to claim 1 wherein
said SPRF comprises less bFGF and/or less G-CSF than PRP and wherein said SPRF comprises less pro-inflammatory factors than PRP said pro-inflammatory factor(s) being selected from the group comprising at least IL-6, IL-8, IL-12, TNF-α.

3. The use of SPRF according to claim 1 or 2 wherein SPRF does not redifferentiate chondrocytes from the dedifferentiated state, preferably if measured by the col II/col I ratio.

4. The use of SPRF according to any of claims 1 to 3 wherein, upon culturing of the chondrocytes *in vitro* said SPRF is applied in the cell culture in a concentration between 1 to 25% or 2-20%, preferably 5 to 15%, highly preferably 8 to 12% or in particular about 10%, wherein the percentage of concentration is given in v/v%

5. A serum fraction of platelet rich fibrin (SPRF), for use in a method for transplantation or implantation of chondrocytes into a patient in need of cartilage repair,
said transplantation or implantation method comprising the steps of
- chondrocytes obtained from a chondrocyte donor subject are contacted with the SPRF,
- the chondrocytes are cultured to proliferate,
- the proliferated chondrocytes are introduced into the patient,
wherein said SPRF is an SPRF prepared from whole blood obtained from an SPRF donor subject by a method as defined in claim 1, and
wherein said SPRF is used for increasing proliferation rate of chondrocytes to be transplanted or implanted to said patient, and
wherein said SPRF provides a lesser extent of redifferentiation of chondrocytes from the dedifferentiated state than platelet-rich plasma (PRP) *in vitro,*
and
wherein SPRF is administered to the patient to the same site as the chondrocytes simultaneous with chondrocyte transplantation.

6. The serum fraction of platelet rich fibrin (SPRF) for use according to claim 5 wherein the patient is a subject in cartilage failure.

7. The SPRF for use according to claim 5 wherein SPRF does not redifferentiate chondrocytes from the dedifferentiated state *in vitro,* if measured by the col II/col I ratio.

8. The SPRF for use according to any of claims 5 to 7 wherein the patient is a subject in need of articular cartilage repair and/or cartilage replacement therapy, preferably articular cartilage repair.

9. The SPRF for use according to any of claims 5 to 8 wherein the patient is a subject having a condition selected from the group consisting of cartilage failure, osteoarthritis, cartilage damage, cellular matrix linkage rupture, chondrocyte protein synthesis inhibition, chondrocyte apoptosis, a condition requiring cartilage regeneration in particular in cartilage ulcer, osteoarthritis or traumatic cartilage loss, a condition requiring subchondral bone regeneration in osteoarthritis, Ahlbeck's disease or osteochondral lesions,
wherein preferably the SPRF is administered to said patient to or at the site of cartilage injury wherein SPRF is contacted with the dedifferentiated chondrocytes also administered to the same site of cartilage injury.

10. The SPRF for use according to any of claims 5 to 9 in an autologous or heterologous transplantation method, wherein said SPRF is an SPRF prepared from whole blood obtained from an SPRF donor subject.

11. The SPRF for use according to any of claims 5 to 10 wherein the SPRF donor subject of the whole blood is identical with the patient.

12. The SPRF for use according to any of claims 5 to 10 wherein the SPRF donor subject is different from the patient, wherein the age of the SPRF donor subject is below 50 years, preferably below 40 years, more preferably below 35 or 30 years.

13. The SPRF for use according to any of claims 5 to 12 wherein SPRF is administered to the patient by injection or by matrix assisted transplantation.

14. The SPRF for use according to any of claims 5 to 13 wherein SPRF is applied in the cell culture in a concentration between 1-25% or 2-20%, preferably 5 to 15%, highly preferably 8 to 12% or in particular about 10%, wherein the percentage of concentration is given in v/v%.

15. A method for increasing proliferation rate of dedifferentiated chondrocytes comprising contacting a serum fraction of platelet rich fibrin (SPRF) with dedifferentiated chondrocytes by adding SPRF to the culturing medium of chondrocytes *in vitro,* thereby improving the proliferation rate of said chondrocytes, said SPRF being prepared from whole blood obtained from one or more donor subject, wherein said SPRF provides a lesser extent of redifferentiation of chondrocytes from the dedifferentiated state than PRP,
wherein said SPRF is obtained by a method as defined in claim 1.

## Patentansprüche

1. Verwendung einer Serumfraktion von plättchenreichem Fibrin (SPRF), hergestellt aus Vollblut das von einem oder mehreren Spendern gewonnen wurde, zur Steigerung der Proliferationsrate dedifferenzierter Chondrozyten *in vitro,*
wobei SPRF im Vergleich zu plättchenreichem Plasma (PRP) eine geringere Redifferenzierung der Chondrozyten aus dem dedifferenzierten Zustand bewirkt,
wobei SPRF ohne exogene Antikoagulanzien durch ein Verfahren gewonnen wird, das folgende Schritte umfasst:
- Bereitstellung von Vollblut, das von einem oder mehreren Spendern gewonnen wurde,
- Zentrifugation des Vollbluts bei 1000 bis 4000 g für 2 bis 20 Minuten innerhalb von 4 Minuten nach der Blutentnahme, wodurch ein Fibrinklumpen (plättchenreiches Fibrin) entsteht, der während der Zentrifugation spontan gerinnt,
- Pressung oder Auspressen des Fibrinklumpens zur Gewinnung des SPRF als Exsudat oder Releasat.

2. Verwendung von SPRF nach Anspruch 1, wobei
das SPRF weniger bFGF und/oder weniger G-CSF als PRP enthält und weniger proinflammatorische Faktoren als PRP enthält, wobei die proinflammatorischen Faktoren aus der Gruppe ausgewählt sind, die mindestens IL-6, IL-8, IL-12 und TNF-α umfasst.

3. Verwendung von SPRF nach Anspruch 1 oder 2, wobei SPRF Chondrozyten nicht aus dem dedifferenzierten Zustand redifferenziert, vorzugsweise gemessen am Verhältnis von Col II zu Col I.

4. Verwendung von SPRF nach einem der Ansprüche 1 bis 3, wobei das SPRF bei der Kultivierung der Chondrozyten *in vitro* in einer Konzentration zwischen 1 und 25 % oder 2 und 20 %, vorzugsweise 5 und 15 %, besonders bevorzugt 8 und 12 % oder insbesondere etwa 10 % zugegeben wird, wobei die Konzentrationsangabe in Vol.-% erfolgt.

5. Eine Serumfraktion von plättchenreichem Fibrin (SPRF) zur Verwendung in einem Verfahren zur Transplantation oder Implantation von Chondrozyten in einen Patienten mit Knorpelreparaturbedarf, wobei dieses Transplantations- oder Implantationsverfahren die folgenden Schritte umfasst:
- Chondrozyten, die von einem Chondrozytenspender gewonnen wurden, werden mit dem SPRF in Kontakt gebracht,
- die Chondrozyten werden zur Proliferation kultiviert,
- die proliferierten Chondrozyten werden dem Patienten zugeführt,
wobei das SPRF ein SPRF ist, das aus Vollblut hergestellt wurde, das von einem SPRF-Spender gemäß einem in Anspruch 1 definierten Verfahren gewonnen wurde, und
wobei das SPRF zur Steigerung der Proliferationsrate der zu transplantierenden oder zu implantierenden Chondrozyten verwendet wird, und
wobei das SPRF *in vitro* eine geringere Redifferenzierung der Chondrozyten aus dem dedifferenzierten Zustand bewirkt als plättchenreiches Plasma (PRP),
und
wobei das SPRF dem Patienten an derselben Stelle verabreicht wird. als die Chondrozyten gleichzeitig mit der Chondrozytentransplantation.

6. Die SPRF zur Verwendung nach Anspruch 5, wobei der Patient an Knorpelschäden leidet.

7. Die SPRF zur Verwendung nach Anspruch 5, wobei die SPRF *in vitro,* gemessen am Col-II/Col-I-Verhältnis, keine Redifferenzierung von Chondrozyten aus dem dedifferenzierten Zustand bewirkt.

8. Die SPRF zur Verwendung nach einem der Ansprüche 5 bis 7, wobei der Patient eine Reparatur und/oder einen Knorpelersatz benötigt, vorzugsweise eine Reparatur des Gelenkknorpels.

9. Das SPRF zur Verwendung nach einem der Ansprüche 5 bis 8, wobei der Patient an einer Erkrankung leidet, die ausgewählt ist aus der Gruppe bestehend aus Knorpelversagen, Arthrose, Knorpelschaden, Bruch der Zellmatrixverbindungen, Hemmung der Chondrozytenproteinsynthese, Chondrozytenapoptose, einer Erkrankung, die eine Knorpelregeneration erfordert, insbesondere bei Knorpelgeschwüren, Arthrose oder traumatischem Knorpelverlust, einer Erkrankung, die eine subchondrale Knochenregeneration erfordert, bei Arthrose, Ahlbeck-Krankheit oder osteochondralen Läsionen,
wobei das SPRF vorzugsweise dem Patienten an der Stelle der Knorpelverletzung verabreicht wird, wo es mit den ebenfalls an derselben Stelle der Knorpelverletzung verabreichten dedifferenzierten Chondrozyten in Kontakt kommt.

10. Das SPRF zur Verwendung nach einem der Ansprüche 5 bis 9 in einem autologen oder heterologen Transplantationsverfahren, wobei es sich bei dem SPRF um ein aus Vollblut eines SPRF-Spenders hergestelltes SPRF handelt.

11. Das SPRF zur Verwendung nach einem der Ansprüche 5 bis 10, wobei der SPRF-Spender des Vollbluts mit dem Patienten identisch ist.

12. Das SPRF zur Verwendung nach einem der Ansprüche 5 bis 10, wobei der SPRF-Spender ein anderer Mensch als der Patient ist und das Alter des SPRF-Spenders unter 50 Jahren, vorzugsweise unter 40 Jahren, besonders bevorzugt unter 35 oder 30 Jahren liegt.

13. Das SPRF zur Verwendung nach einem der Ansprüche 5 bis 12, wobei das SPRF dem Patienten durch Injektion oder matrixgestützte Transplantation verabreicht wird.

14. Das SPRF zur Verwendung nach einem der Ansprüche 5 bis 13, wobei das SPRF in der Zellkultur in einer Konzentration zwischen 1 und 25 % oder 2 und 20 %, vorzugsweise 5 bis 15 %, besonders bevorzugt 8 bis 12 % oder insbesondere etwa 10 % angewendet wird, wobei die Konzentrationsangabe in Vol.-% erfolgt.

15. Verfahren zur Steigerung der Proliferationsrate von dedifferenzierten Chondrozyten, umfassend das Inkontaktbringen einer Serumfraktion von plättchenreichem Fibrin (SPRF) mit dedifferenzierten Chondrozyten durch Zugabe von SPRF zum Kulturmedium von Chondrozyten *in vitro,* wodurch die Proliferationsrate der Chondrozyten verbessert wird, wobei das SPRF aus Vollblut hergestellt wird, das von einem oder mehreren Spendern gewonnen wurde, wobei das SPRF eine geringere Redifferenzierung der Chondrozyten aus dem dedifferenzierten Zustand bewirkt als PRP,
wobei das SPRF durch ein Verfahren gemäß Anspruch 1 gewonnen wird.

## Revendications

1. Utilisation d'une fraction de sérum de fibrine riche en plaquettes (SPRF) préparée à partir de sang total obtenu auprès d'un ou plusieurs sujet(s) donneur(s), pour augmenter le taux de prolifération de chondrocytes dédifférenciés *in vitro,*
dans laquelle ladite SPRF fournit un moindre degré de redifférenciation des chondrocytes à partir de l'état dédifférencié que le plasma riche en plaquettes (PRP), dans laquelle
ladite SPRF est obtenue, sans anticoagulants exogènes, par un procédé comprenant les étapes de
- fournir du sang total obtenu auprès du ou des sujet(s) donneur(s),
- centrifuger le sang total à 1000 à 4000 g pendant 2 à 20 minutes dans les 4 minutes suivant le prélèvement sanguin, fournissant ainsi un caillot de fibrine (fibrine riche en plaquettes), qui coagule spontanément lors de sa préparation par centrifugation,
- presser ou comprimer le caillot de fibrine pour obtenir la SPRF sous forme d'exsudat ou de produit de libération de celui-ci.

2. Utilisation de la SPRF selon la revendication 1, dans laquelle
ladite SPRF comprend moins de bFGF et/ou moins de G-CSF que le PRP et dans laquelle ladite SPRF comprend moins de facteurs pro-inflammatoires que le PRP, ledit ou lesdits facteur(s) pro-inflammatoire(s) étant choisi(s) dans le groupe comprenant au moins IL-6, IL-8, IL-12, TNF-α.

3. Utilisation de la SPRF selon la revendication 1 ou 2, dans laquelle la SPRF ne redifférencie pas les chondrocytes à partir de l'état dédifférencié, de préférence s'il est mesuré par le rapport col II/col I.

4. Utilisation de la SPRF selon l'une quelconque des revendications 1 à 3, dans laquelle, lors de la culture des chondrocytes *in vitro,* ladite SPRF est appliquée dans la culture cellulaire à une concentration comprise entre 1 et 25 % ou 2 à 20 %, de préférence entre 5 et 15 %, de manière hautement préférée entre 8 et 12 % ou en particulier environ 10 %, le pourcentage de concentration étant exprimé en % v/v.

5. Une fraction de sérum de fibrine riche en plaquettes (SPRF), pour une utilisation dans un procédé de transplantation ou d'implantation de chondrocytes chez un patient ayant besoin d'une réparation du cartilage,
ledit procédé de transplantation ou d'implantation comprenant les étapes de
- des chondrocytes obtenus à partir d'un sujet donneur de chondrocytes sont mis en contact avec la SPRF,
- les chondrocytes sont cultivés pour proliférer,
- les chondrocytes proliférés sont introduits dans le patient,
dans laquelle ladite SPRF est une SPRF préparée à partir de sang total obtenu auprès d'un sujet donneur de SPRF par un procédé tel que défini dans la revendication 1, et
dans laquelle ladite SPRF est utilisée pour augmenter le taux de prolifération de chondrocytes à transplanter ou à implanter chez ledit patient, et
dans laquelle ladite SPRF fournit un moindre degré de redifférenciation des chondrocytes à partir de l'état dédifférencié que le plasma riche en plaquettes (PRP) *in vitro,*
et
dans laquelle la SPRF est administrée au patient au même site que les chondrocytes simultanément à la transplantation de chondrocytes.

6. La fraction de sérum de fibrine riche en plaquettes (SPRF) pour une utilisation selon la revendication 5, dans laquelle le patient est un sujet présentant une défaillance cartilagineuse.

7. La SPRF pour une utilisation selon la revendication 5, dans laquelle la SPRF ne redifférencie pas les chondrocytes à partir de l'état dédifférencié *in vitro,* s'il est mesuré par le rapport col II/col I.

8. La SPRF pour une utilisation selon l'une quelconque des revendications 5 à 7, dans laquelle le patient est un sujet ayant besoin d'une réparation du cartilage articulaire et/ou d'une thérapie de remplacement du cartilage, de préférence d'une réparation du cartilage articulaire.

9. La SPRF pour une utilisation selon l'une quelconque des revendications 5 à 8, dans laquelle le patient est un sujet présentant une affection choisie dans le groupe constitué par une défaillance cartilagineuse, une arthrose, une lésion du cartilage, une rupture de liaisons de la matrice cellulaire, une inhibition de la synthèse des protéines chondrocytaires, une apoptose des chondrocytes, une affection nécessitant une régénération du cartilage en particulier dans un ulcère du cartilage, une arthrose ou une perte traumatique du cartilage, une affection nécessitant une régénération osseuse sous-chondrale dans l'arthrose, la maladie d'Ahlbeck ou des lésions ostéochondrales,
dans laquelle, de préférence, la SPRF est administrée audit patient vers ou sur le site de lésion du cartilage, la SPRF étant mise en contact avec les chondrocytes dédifférenciés également administrés au même site de lésion du cartilage.

10. La SPRF pour une utilisation selon l'une quelconque des revendications 5 à 9 dans un procédé de transplantation autologue ou hétérologue, dans laquelle ladite SPRF est une SPRF préparée à partir de sang total obtenu auprès d'un sujet donneur de SPRF.

11. La SPRF pour une utilisation selon l'une quelconque des revendications 5 à 10, dans laquelle le sujet donneur de SPRF du sang total est identique au patient.

12. La SPRF pour une utilisation selon l'une quelconque des revendications 5 à 10, dans laquelle le sujet donneur de SPRF est différent du patient, dans laquelle l'âge du sujet donneur de SPRF est inférieur à 50 ans, de préférence inférieur à 40 ans, plus préférentiellement inférieur à 35 ou 30 ans.

13. La SPRF pour une utilisation selon l'une quelconque des revendications 5 à 12, dans laquelle la SPRF est administrée au patient par injection ou par transplantation assistée par matrice.

14. La SPRF pour une utilisation selon l'une quelconque des revendications 5 à 13, dans laquelle la SPRF est appliquée dans la culture cellulaire à une concentration comprise entre 1 et 25 % ou 2 à 20 %, de préférence entre 5 et 15 %, de manière hautement préférée entre 8 et 12 % ou en particulier environ 10 %, le pourcentage de concentration étant exprimé en % v/v.

15. Procédé pour augmenter le taux de prolifération de chondrocytes dédifférenciés comprenant la mise en contact d'une fraction de sérum de fibrine riche en plaquettes (SPRF) avec des chondrocytes dédifférenciés en ajoutant de la SPRF au milieu de culture de chondrocytes *in vitro,* améliorant ainsi le taux de prolifération desdits chondrocytes, ladite SPRF étant préparée à partir de sang total obtenu auprès d'un ou plusieurs sujet(s) donneur(s), dans lequel ladite SPRF fournit un moindre degré de redifférenciation des chondrocytes à partir de l'état dédifférencié que le PRP,
dans lequel ladite SPRF est obtenue par un procédé tel que défini dans la revendication 1.
